# EUROPÄISCHE PATENTSCHRIFT

(11) **EP 3 454 802 B1**
(45) Veröffentlichungstag und Bekanntmachung des Hinweises auf die Patenterteilung: **12.10.2022**
(21) Anmeldenummer: 17722446.6
(22) Anmeldetag: 09.05.2017
(51) Int. Cl.: A61F 9/008

(54) **PLANUNGSEINRICHTUNG UND VERFAHREN ZUM ERZEUGEN VON STEUERDATEN FÜR EINE BEHANDLUNGSVORRICHTUNG ZUR AUGENCHIRURGIE**
PLANNING DEVICE AND METHOD FOR GENERATING CONTROL DATA FOR AN OPHTHALMIC SURGERY DEVICE
DISPOSITIF DE PLANIFICATION ET PROCEDE DE GENERATION DE DONNEES DE COMMANDE POUR UN DISPOSITIF DE CHIRURGIE OPHTALMIQUE

(30) Priorität: 10.05.2016 DE 102016208011
(43) Veröffentlichungstag der Anmeldung: 20.03.2019
(73) Patentinhaber: Carl Zeiss Meditec AG, 07745 Jena (DE)
(72) Erfinder: KINDT, Johannes, 99425 Weimar (DE); DICK, Manfred, 07926 Gefell (DE); BISCHOFF, Mark, 07749 Jena (DE)
(74) Vertreter: Beck, Bernard
(86) Internationale Anmeldenummer: PCT/EP2017/061101
(87) Internationale Veröffentlichungsnummer: WO 2017/194566

(56) Entgegenhaltungen:
- DE-A1-102007 019 813
- US-A1- 2015 335 477
- US-B2- 8 486 055

## Beschreibung

Die Erfindung bezieht sich auf eine Planungseinrichtung zum Erzeugen von Steuerdaten für eine Behandlungsvorrichtung, die mittels einer Lasereinrichtung zumindest eine Schnittfläche in der Hornhaut erzeugt. Die Erfindung bezieht sich weiter auf eine Behandlungsvorrichtung, die eine Planungseinrichtung der genannten Art aufweist.

Die Erfindung bezieht sich weiter auf ein Verfahren zum Erzeugen von Steuerdaten für eine Behandlungsvorrichtung, die mittels einer Lasereinrichtung zumindest eine Schnittfläche in der Hornhaut erzeugt. Es wird ein Verfahren zur Augenchirurgie beschrieben, welches jedoch ganz explizit nicht Teil der Erfindung ist, wobei mittels einer Behandlungsvorrichtung mit einer Lasereinrichtung zumindest eine Schnittfläche in der Hornhaut erzeugt wird.

Im Stand der Technik sind verschiedenste Behandlungsverfahren mit dem Ziel der Refraktionskorrektur am menschlichen Auge bekannt. Ziel der Operationsmethoden ist es dabei, die Hornhaut gezielt zu verändern, um so die Lichtbrechung im Auge zu beeinflussen. Hierfür werden mehrere Operationsmethoden eingesetzt. Die verbreitetste Methode ist die sogenannte Laser-Insitu-Keratomileusis, die auch LASIK abgekürzt wird. Dabei wird eine Hornhaut-Lamelle von der Hornhautoberfläche einseitig gelöst und zur Seite geklappt. Das Lösen dieser Lamelle kann mittels eines mechanischen Mikrokeratoms erfolgen, oder auch mittels eines sogenannten Femtosekunden-Laserkeratoms, wie es z.B. von Intralase Corp., Irvine, USA, vertrieben wird. Nachdem die Lamelle gelöst und zur Seite geklappt wurde, ist bei der LASIK- Operation die Anwendung eines Excimer-Lasers vorgesehen, der das derart unter der Lamelle freigelegte Hornhautgewebe durch Ablation abträgt. Nachdem auf diese Art und Weise Hornhautgewebe oberflächlich verdampft wurde, welches ursprünglich unter der Hornhautoberfläche lag, wird die Hornhaut-Lamelle wieder auf den ursprünglichen Platz zurückgeklappt.

Die Anwendung eines Laserkeratoms zum Freilegen der Lamelle ist gegenüber einem mechanischen Messer vorteilhaft, da die die geometrische Präzision verbessert und die Häufigkeit klinisch relevanter Komplikationen verringert ist. Insbesondere kann die Lamelle mit sehr viel konstanterer Dicke hergestellt werden, wenn Laserstrahlung verwendet wird. Auch ist die Schnittkannte präzise geformt, was die Gefahr für Heilungsstörungen durch diese auch nach der Operation verbleibende Grenzfläche mindert. Nachteilig bei diesem Verfahren ist allerdings, dass zwei unterschiedliche Behandlungsvorrichtungen verwendet werden müssen, zum einen nämlich das Laserkeratom zum Freilegen der Lamelle und zum anderen der das Hornhautgewebe verdampfende Laser.

Diese Nachteile sind behoben bei einem Verfahren, das jüngst durch die Carl Zeiss Meditec AG implementiert wurde und mit der Bezeichnung FLEx (Femtosecond Lenticule EXtraction) abgekürzt wird. Bei diesem Verfahren zur Lentikelextraktion wird mittels eines Kurzpulslasers, vorzugsweise eines Femtosekundenlasers in der Augenhornhaut eine Schnittgeometrie gebildet, welche in der Hornhaut ein Hornhaut-Volumen (sog. Lentikel) separiert. Dieses wird dann manuell vom Operateur entnommen, nachdem die das Lentikel bedeckende Lamelle zur Seite geklappt wurde. Der Vorteil dieses Verfahrens liegt zum einen darin, dass die Schnittqualität durch Anwendung des Femtosekundenlasers kombiniert mit einem gekrümmten Kontaktglas nochmals verbessert ist.

Zum anderen ist nur noch eine Behandlungsvorrichtung erforderlich; der Excimer-Laser wird nicht mehr eingesetzt. Diese Methode vermeidet auch Risiken und Beschränkungen des Excimer-Lasers.

Eine Weiterentwicklung des FLEx-Verfahrens wird in der Literatur heute als SMILE-Verfahren bezeichnet, bei dem kein Flap erzeugt wird, sondern nur ein kleiner Öffnungsschnitt als Zugang zu dem unter dem sogenannten Cap liegenden Lentikel dient. Das separierte Lentikel wird durch diesen kleinen Öffnungsschnitt entnommen, wodurch die biomechanische Integrität der vorderen Hornhaut weniger beeinträchtigt wird als bei LASIK, oder ähnlichen Methoden. Hinzu kommt, dass auf diese Weise oberflächliche weniger Nervenfasern in der Hornhaut zerschnitten werden, was sich wahrscheinlich vorteilhaft auf die Wiederherstellung der ursprünglichen Sensibilität der Hornhautoberfläche auswirkt. Das nach LASIK oft zu behandelnde Symptom trockener Augen ist dadurch in seiner Ausprägung und Dauer reduziert. Auch andere Komplikationen nach LASIK, die meist mit dem Flap im Zusammenhang stehen (z.B. Flap-Verschiebung, Falten, Epithel-Einwachsungen im Flapbett) treten ohne Flap seltener auf. Eine Vorrichtung und entsprechende Steuerung sind in DE 10 2007 019 813 beschrieben.

Bei der Erzeugung von Schnittflächen in der Hornhaut mittels Laserstrahlung wird üblicherweise die optische Strahlungswirkung dadurch ausgenutzt, dass ein optischer Durchbruch durch einzelne optische Pulse, deren Dauer zwischen etwa 100 fs und 100 ns liegen kann, erzeugt wird. Auch ist es bekannt, einzelne Pulse, deren Energie unter einem Schwellwert für einen optischen Durchbruch liegt, derart überdeckt ins Gewebe bzw. Material einzubringen, dass auch damit eine Material- bzw. Gewebetrennung erreicht wird. Dieses Konzept der Schnitterzeugung im Hornhautgewebe erlaubt eine große Vielfalt an Schnitten.

Es ist weiterhin bekannt, dass korneales Gewebe mittels Laserstrahlung so verändert werden kann, dass es zwar seine Transparenz beibehält, jedoch eine lokale Brechkraftänderung erfolgt (DE 41 31 361, DE 199 43 723 und DE 199 43 735 der Anmelderin, US 8 486 055 Knox et al). Es ist zudem bekannt, diesen Effekt zu nutzen, um diffraktive Strukturen zu erzeugen, welche die Abbildungseigenschaften des gesamten Auges so verändern, dass eine bestehende Fehlsichtigkeit ausgeglichen werden kann. Dabei kann die Brechkraftänderung durch Änderung des lokalen Brechungsindexes des Hornhautmaterials, aber auch durch mechanische Änderung (z.B. Crosslinking der Kollagenfasern der Hornhaut) bewirkt werden. Diese Veränderungen können sowohl bewirkt werden durch direkte Wechselwirkung der Laserstrahlung mit dem Gewebe bei einer Laserleistung unterhalb der Schwelle bei der ein Durchbruch erzeugt wird, als auch durch Wechselwirkung der Laserstrahlung mit in die Hornhaut eingebrachten Stoffen. Als Stoffe können beispielsweise Photosensitizer (z.B. Riboflavin), IOP-reduzierende Pharmazeutika (IOP = Intraocular Pressure = Augeninnendruck), Antimykotika, Antibiotika, Stammzellen oder Nanopartikel zur Anwendung kommen. Solche Verfahren werden auch als LIRIC (laser induced refractive index change) bezeichnet. LIRIC erfolgt insbesondere derart, dass Laserstrahlung in das Bearbeitungsgebiet fokussiert wird und dort zu einer Strukturveränderung des Materials führt, die dauerhaft bestehen bleibt, das Material transparent erhält und die lokale Brechzahl des Materials und damit die Brechkraft des transparenten Gewebes (Hornhaut, Linse) verändert. Ein Vorteil dieses Verfahren könnte sein, dass zur Fehlsichtigkeitskorrektur keine Gewebetrennungen in der Hornhaut vorgenommen werden müssen. Andererseits ist eine sehr hohe Genauigkeit der Positionierung des Laserfokus notwendig, wozu im Allgemeinen das Auge mittels Ansaugen an ein Kontaktelement fixiert werden muss.

Aber auch bei der Durchführung des LIRIC-Verfahrens kann es zu Fehlern kommen, insbesondere möglich sind:
- Planungsfehler
- Bearbeitungsfehler durch
   ∘ Zentrierfehler
   ∘ Verrutschen durch fehlende, unterbrochene oder nicht ausreichende Fixierung
   ∘ Behandlungsabbruch durch Unterbrechung der Fixierung
   ∘ Behandlungsabbruch durch technisches Versagen der Vorrichtung
- Klinische Nebenwirkungen (z.B. Zunahme der Streuung oder Absorption)
- Regression der Korrektur
- Progression der Fehlsichtigkeit
- Unzufriedenheit des Patienten mit dem Ergebnis der Behandlung

Das LIRIC-Verfahren kann theoretisch auch zur Korrektur des Behandlungsergebnisses verwendet werden, aber das erfordert eine präzise Positionierung der Zweitbehandlung in Bezug auf die Erstbehandlung (die Erstbehandlung endet mit dem Lösen der Fixation). Wenn die Erstbehandlung selbst (in sich) bereits Fehlpositionierungen aufweist, weil es beispielsweise zum Verrutschen gekommen ist, so kann eine Reparatur sehr aufwendig werden. Eventuell muss dann die erzeugte Beugungsstruktur unter Ausnutzung der Sättigung des Bearbeitungseffekts "gelöscht" werden, indem auch unbearbeitete Regionen bearbeitet werden. Dabei bleibt im besten Fall ein komplett bearbeitetes Gebiet ohne die beabsichtigte refraktive Wirkung übrig, welches allerdings verstärkte Nebenwirkungen wie Streuung usw. verursachen kann.

Der Erfindung liegt deshalb die Aufgabe zugrunde, eine Planungseinrichtung zum Erzeugen von Steuerdaten, eine Behandlungsvorrichtung zur Refraktion korrigierenden Augenchirurgie sowie ein Verfahren zum Erzeugen von Steuerdaten für eine solche Behandlungsvorrichtung anzugeben, welche in der Lage ist, eine mittels LIRIC erzeugte Struktur, welche eine unbeabsichtigter Wirkung verursacht, so zu verändern, dass die unbeabsichtigte Wirkung verschwindet. Vorteilhaft wäre es, wenn die ursprünglich beabsichtigte Wirkung der LIRIC-Struktur gleichzeitig oder nachfolgend erzielt würde.

Diese Aufgabe wird erfindungsgemäß mit einer Planungseinrichtung der eingangs genannten Art gelöst, die Berechnungsmittel zum Festlegen von Hornhaut-Schnittflächen aufweist, wobei die Berechnungsmittel die Schnittflächen so bestimmen, dass ein Lentikel gebildet wird, welches die mittels LIRIC erzeugte Struktur räumlich umfasst (umschreibt, einschließt), so dass diese mit der Entfernung des Lentikels aus der Hornhaut ebenfalls komplett aus der Hornhaut entfernt werden kann.

Die Erfindung wird weiter gelöst mit einer Behandlungsvorrichtung, die eine Lasereinrichtung aufweist, welche mittels Laserstrahlung gemäß Steuerdaten zumindest eine Schnittfläche in der Hornhaut trennt, und eine Planungseinrichtung nach der soeben genannten Art zum Erzeugen der Steuerdaten aufweist, wobei die Planungseinrichtung die Schnittflächen so bestimmt, dass ein Lentikel gebildet wird, welches die mittels LIRIC erzeugte Struktur umschreibt, so dass diese komplett aus der Hornhaut entfernt werden kann.

Die Erfindung wird schließlich ebenfalls gelöst mit einem Verfahren zum Erzeugen von Steuerdaten gemäß der eingangs genannten Art, das aufweist: Erzeugen eines Steuerdatensatzes für die Hornhaut-Schnittfläche zur Ansteuerung der Lasereinrichtung, wobei die Planungseinrichtung die Schnittflächen so bestimmt, dass ein Lentikel gebildet wird, welches die mittels LIRIC erzeugte Struktur umschreibt, so dass diese komplett aus der Hornhaut entfernt werden kann.

Dabei kann insbesondere das Lentikel so geformt sein, dass sich nach seiner Entfernung aus der Hornhaut die ursprüngliche beabsichtigte Fehlsichtigkeitskorrektur einstellt, beispielsweise durch entsprechende Änderung der Krümmung der Hornhautvorderseite.

Es kann vorteilhaft sein, wenn Schnitte für das Lentikel bereits vor der Durchführung des LIRIC-Verfahrens angelegt werden, alternativ können sie aber auch erst später bei bestehender Notwendigkeit eingebracht werden.

Der Stand der Technik zur Extraktion eines Lentikels (mit einer definierten Brechkraft) wird genutzt zur Extraktion eines speziellen Lentikels (welches im Folgenden zur besseren Unterscheidung vom "klassischen" Lentikel zum Zweck der Brechkraftkorrektur als Enhancement-Lentikel bezeichnet wird) zur Entfernung einer unerwünschten LIRIC-Struktur.

Ein Enhancement-Lentikel kann erzeugt werden, welches die zu entfernende LIRIC-Struktur vollständig einschließt.

Durch die Entfernung des Enhancement-Lentikels wird also die LIRIC-Struktur im gewünschten Umfang aus dem Auge entfernt. Dies erfolgt zum Zweck der erneuten Änderung der optischen Eigenschaften des Auges.

Um ein hohes Maß an Sicherheit für den Patienten zu erreichen, wird die Form des Enhancement-Lentikels so gewählt, dass seine Entfernung nicht zu einem inakzeptablen refraktiven Ergebnis der Behandlung führen würde. Das Enhancement-Lentikel kann also ohne großen Aufwand für den Anwender operativ entfernt werden, um auf diese Weise die LIRIC-Struktur aus dem Auge zu entfernen und gleichzeitig kann die Entfernung eine günstige (oder zumindest akzeptable) refraktive Wirkung erzielen. Insbesondere kann die Form des Enhancement -Lentikels zur refraktiven Korrektur eines manifesten Sehfehlers des Auges mittels Lentikelextraktion (SMILE) angepasst sein. In Spezialfällen sind aber auch Enhancement-Lentikel mit einer solchen Geometrie möglich, die gerade keine formbedingte refraktive Änderung bewirken. Diese weisen eine ungefähr konstante Dicke auf, jedoch besitzen sie einen gewissen Profilverlauf, der die Brechkraft der Hornhaut bei ihrer Entfernung gerade nicht nicht wesentlich ändert.

Die Erzeugung eines Enhancement-Lentikels erfolgt vorteilhaft zumindest teilweise bereits im Zuge der LIRIC-Behandlung: präventives Enhancement-Lentikel. Beispielsweise kann der Lentikelschnitt bereits posterior bezüglich der LIRIC-Struktur eingebracht werden bevor die LIRIC-Struktur das Gewebe verändert. Es kann zusätzlich auch bereits der Cap-Schnitt bzw. ein Flap-Schnitt anterior zur RIS-Struktur eingebracht werden. Der Vorteil dieses Verfahrens , welches per se nicht Teil der Erfindung ist, besteht darin, dass somit ein hohes Maß an Sicherheit hinsichtlich der relativen Lage der Strukturen besteht und eine Beeinflussung der Schnittführung durch LIRIC-Strukturen von vornherein ausgeschlossen wird. In einer allgemeineren Ausführung wird die Erzeugung einer LIRIC-Struktur in Kombination mit mindestens einem Schnitt in der Hornhaut beschrieben. Falls statt Lentikelextraktion ein ablatierendes Verfahren zur Entfernung der defekten LIRIC-Struktur benutzt werden soll, kann es vorteilhaft sein, bereits im Zusammenhang mit der RIS-Behandlung einen präventiven Enhancement-Schnitt einzubringen, der insbesondere ein Flap-schnitt sein kann.

Die Erfindung besteht in allgemeinster Form in einer Planungseinrichtung zum Erzeugen von Steuerdaten für eine Behandlungsvorrichtung und einem Verfahren dieser Erzeugung von Steuerdaten um eine präventive Enhancement-Struktur (PES) zu erzeugen, die für die primäre Behandlung nicht notwendig ist, allerdings im Fall einer Zweitbehandlung deren Durchführung erleichtert oder ihre Wirkung verbessert. Diese Enhancement-Struktur kann eine der oben bereits beschriebenen Ausprägungen haben, kann beispielsweise aber auch nur eine Markierung sein, die dazu dient, die relative Lage einer Zweitbehandlung bezüglich der Erstbehandlung zu bestimmen.

Die Enhancement-Struktur kann auch erst nach Einbringung der LIRIC-Struktur erzeugt werden. Damit ist neben dem generellen Problem der richtigen Positionierung der Zweitbehandlung bezüglich der Erstbehandlung insbesondere das Problem verbunden, dass sich die LIRIC-Struktur auf die Herstellung der Enhancement-Struktur störend auswirken könnte. Dies kann bei der Schnittführung berücksichtigt werden, indem eine Pre-Kompensation der Wirkung der LIRIC-Struktur derart erfolgt, dass im Zusammenwirken der Zweitbehandlung mit der vorhandenen Wirkung der Erstbehandlung gerade der beabsichtigte Bearbeitungseffekt eintritt. Das ist unter Umständen jedoch aufwändiger als die präventive Erzeugung von Enhancement-Strukturen.

Neben der zeitlich vor- oder nachgeschalteten Einbringung der Enhancement-Struktur in Bezug zur LIRIC-Struktur ist vorgesehen in einer kombinierten Behandlung die erforderlichen Behandlungsschritte in einer räumlichen Abfolge von der Rückseite der Hornhaut zur Vorderseite der Hornhaut durchzuführen. Dabei ist sichergestellt, dass im kornealen Gewebe immer durch ein unbehandeltes Gebiet hindurch die Ultrakurzpuls-Laserstrahlung eingebracht/fokussiert werden kann, ohne optische Effekte einer vorab durchgeführten Behandlung berücksichtigen zu müssen.

Dabei kann dieses Verfahren auf ein universelles Lasersystem zurückgreifen, welches die LIRIC- Behandlung durchführen als auch die Enhancement-Struktur herstellen kann. So kann beispielsweise die LIRIC-Behandlung mit einem Ultrakurzpulslasersystem mit 405 nm Wellenlänge durchgeführt werden, welches gleichzeitig in einer weiteren Betriebsart in der Lage ist, das korneale Gewebe zu perforieren und die Enhancement Struktur einzubringen. Weiterhin kann ein Lasersystem mit einer für eine LIRIC-Behandlung notwendigen Wellenlänge im kurzwelligen Spektralbereich von ca. 300 - 450 nm mit einem weiteren Wellenlängenbereich von ca. 800nm - 1070nm für die Einbringung der Enhancement Struktur bereitgestellt werden. Dieses Lasersystem weist beispielsweise eine umschaltbare Frequenzvervielfachung wie Frequenzverdopplung (SHG - Second Harmonic Generation) oder THG (Third Harmonic Generation) auf. Weiterhin ist es ebenfalls möglich, die Behandlungen auf unterschiedlichen (Laser-)Geräten durchzuführen, welche über plattformübergreifend definierte Protokolle die LIRIC-Struktur und die Enhancement-Struktur zueinander positionieren können.

Eine weitere wünschenswerte Variante ist es, ein Enhancement-Lentikel relativ zu einer bereits vorhandenen LIRIC-Struktur positionieren zu können, deren genaue Lage aber unbekannt ist (z.B. aufgrund eines Fehlers bei einer früheren LIRIC-Behandlung). Die Lage der LIRIC-Struktur in der Hornhaut wird hierzu gemessen. Zur Beobachtung und Lokalisierung der LIRIC-Struktur kommen Phasenkontrastmikroskopie oder OCT (Optische Kohärenztomographie) von Strukturen nahe hinter der LIRIC-Struktur in Frage (Korneales Stroma, Endothelzellen, Membranen), oder Konfokal- oder Widefield- Fluoreszenzmikroskopie des durch LIRIC veränderten Stromas. Scannende Mikroskopieverfahren können sich dabei desselben Scanners bedienen wie das Behandlungssystem für das SMILE- bzw. LIRIC-Verfahren.

Neben der Vorbereitung einer Enhancement-Struktur zur nahezu nebenwirkungsfreien Entfernung einer LIRIC-Struktur ist es ebenfalls vorgesehen, eine intraoperative Dosimetrie der individuell erzeugten LIRIC-Struktur einzuführen. Diese beruht beispielsweise darauf, dass vor und nach Einbringung einer lokalen LIRIC-Struktur eine der lokalen Struktur zugeordnete optische Pachymetrie der Kornea erfolgt, die eine Brechzahl und/oder Dickenänderung der Hornhaut an dieser Stelle vermessen kann. Da diese dann sequentiell registrierten Messungen einen Relativwert ermitteln welcher dem LIRIC-Effekt zugeordnet ist, kann individuell eine sehr hohe Messgenauigkeit des LIRIC-Effektes durch diese Pachymetrie erfolgen. Neben einer prä - und postoperativen pachymetrischen Dosimetrie ist eine intraoperative Dosimetrie vorgesehen. Vorzugsweise ist eine Pachymetrie mit einem hochauflösendem Verfahren der Optischen Kohärenztomografie (OCT) vorgesehen. Dabei wird der punktweise A-scan als Punkt-Pachymetrie genutzt und die laterale B-Scan Auflösung zur räumlichen 3-D Dosimetrie genutzt. Eine weitere Möglichkeit zur Dosimetrie ist die ortsaufgelöste Messung der Autofluoreszenz der Hornhaut, welche durch die Behandlung angeregt wird, oder welche in einem dem Behandlungsfokus folgenden Messfokus mit einer weiteren Lichtquelle angeregt wird.

Da LIRIC ein rasterndes Verfahren ist, und aufeinanderfolgende Behandlungsfoki eng beieinander liegen (z.B. 0,5-5µm), zwischen denen nur kleine Änderungen der Gewebeeigenschaften zu erwarten sind, können die Ergebnisse der Onlinemessung auch prädiktiv für die Korrektur der Behandlungsstärke der jeweils folgenden Behandlungsfoki verwendet werden.

Mittels dieser Dosimetrie kann somit online die individuelle Behandlung optimiert werden und objektiv ein Behandlungsergebnis dokumentiert werden.

Zur Durchführung des Verfahrens ist eine Vorrichtung vorgesehen, welche die einige oder alle der folgende Aufgaben beispielsweise mit den angegebenen Mitteln erfüllen kann:
1. Struktur berechnen: die Berechnung einer LIRIC-Struktur ist nicht Gegenstand dieser Erfindung, eine Beschreibung findet sich in US 8 486 055, auf deren Inhalt hiermit Bezug genommen wird. Die Berechnung einer Lentikelform und die Ableitung entsprechender Steuersignale ist bspw. aus DE 10 2009 005 482 bekannt, auf deren Inhalt hiermit Bezug genommen wird. Dafür sind vorgesehen: Eingabemittel für die zur Berechnung notwendigen Größen, Mittel zur Speicherung und Berechnung der gewünschten Lentikelgeometrie.
2. Struktur lagerichtig einbringen: Zentrierung und Navigation sind Aufgaben, die bereits im Zusammenhang mit anderen refraktiven Behandlungen gelöst sind und daher an sich bekannt. Insbesondere das lagerichtige Fixieren eine Patientenauges an einem Vakuumfixationselement ist wichtig. Die Verwendung eines Kontaktglases ist vorteilhaft aber nicht zwingend. Auch ein Liquid-Interface kann verwendet werden. Dafür sind vorgesehen ein entsprechendes laseroptisches System, Mittel zur Kalibrierung des Systems, Detektion der relevanten Strukturen, Tracking der Laserfoki, OCT für die Kalibrierung der Transformation auf die durch das Fixationselement veränderte Augengeometrie (Analyse der Verzerrung der Pre-Op-Pachymetrie und Einbeziehung in die Planung für die Behandlungsfoki).
3. Koordinatensysteme definieren und aufeinander referenzieren: Diese Lösung für diese Aufgabe ist für die Erzeugung von Lentikeln oder die Navigation bei Verwendung von Kontaktgläsern (Kontaktglasdetektion) und auch von Liquid-Interfaces bereits bekannt (bspw. aus DE 10 2009 005 482). Die relative Lagebeziehung zwischen LIRIC-Struktur und Enhancement-Lentikel (allg. Enhancement-Struktur oder präventive Enhancement-Struktur) kann durch die Erfindung einfach sichergestellt werden, wenn die richtige Lagebeziehung durch gleichzeitiges Einbringen der LIRIC-Strukur und der präventive Enhancement-Struktur hergestellt wird.
4. Registrierung durch Erzeugung und Verarbeitung von Registrierdaten.
5. Deformationstransformationen: Transformation auf die durch das Fixationselement veränderte Augengeometrie durch Berücksichtigung der Kontaktglasgeometrie und natürliche Augengeometrie. Dafür sind vorgesehen: Mittel zur Speicherung, Berechnung.
6. Überwachung der Bearbeitung: Beleuchtung (im Infraroten und oder visuellen Spektralbereich) und Beobachtung, OCT, evtl. Konfokale Detektion (vgl. DE 103 23 422)
7. Detektion: Mitgeführte Detektion indem Teilstrahlen zur Referenzierung genutzt werden (bspw. Phasenkontrastmikroskopie)
8. Logging: inkrementelle Fortschrittsprotokollierung
9. Sicherheitsfunktionen: Dafür sind vorgesehen: Leistungsregelung, Referenzmarkenerzeugung, Sicherheitsabschaltung.
10. Unterbrochene Bearbeitung wieder aufnehmen: Definition von Kompensationsschreiblagen für die Wiederaufnahme der Bearbeitung nach einem ggf. notwendigen Abbruch.
11. Log-Information auswerten: bei Wiederaufnahme die vorhandene Information über Erstbehandlung in die Behandlungsplanung einbeziehen
12. Detektion: Post-Op-Pachymetrie vs. Pre-Op-Pachymetrie verwenden, um die Wellenfrontänderung intraoperativ zu messen, Streumessungen an der brechenden Struktur
13. Zweitbearbeitung lagerichtig einbringen durch Auswertung von Referenzmarken
14. Information über primäre Bearbeitung verarbeiten: Iterative Erzeugung der Gesamtwirkung, wobei die Teilwirkung vorangegangener Bearbeitungsphasen zur Planung der nachfolgenden Phase verwendet werden (Bearbeitungsphase kann dabei ein einzelner Behandlungsfokus, eine Zeile, oder ein Teilgebiet von Behandlungsfoki sein)
15. Detektion: Streumessung an der LIRIC-Struktur

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, noch näher erläutert. Es zeigen:
Fig. 1 eine schematische Darstellung einer Behandlungsvorrichtung mit einer Planungseinrichtung für eine Behandlung bei augenchirurgischer Refraktionskorrektur,
Fig. 2 eine schematische Darstellung der Wirkung der Laserstrahlung, die in der Behandlungsvorrichtung der Fig. 1 verwendet wird,
Fig. 3 eine weitere Schemadarstellung des Behandlungsgerätes der Fig. 1 hinsichtlich der Einbringung der Laserstrahlung,
Fig. 4 eine schematische Schnittdarstellung durch die Augenhornhaut zur Veranschaulichung der Entnahme des Hornhaut-Volumens im Zusammenhang mit der augenchirurgischen Refraktionskorrektur,
Fig. 5 eine Schemadarstellung hinsichtlich des Aufbaus des Behandlungsgerätes der Fig. 1 mit besonderem Bezug auf die dort vorhandene Planungseinrichtung,
Fig. 6 eine Schemadarstellung einer erfindungsgemäßen Lentikelgeometrie

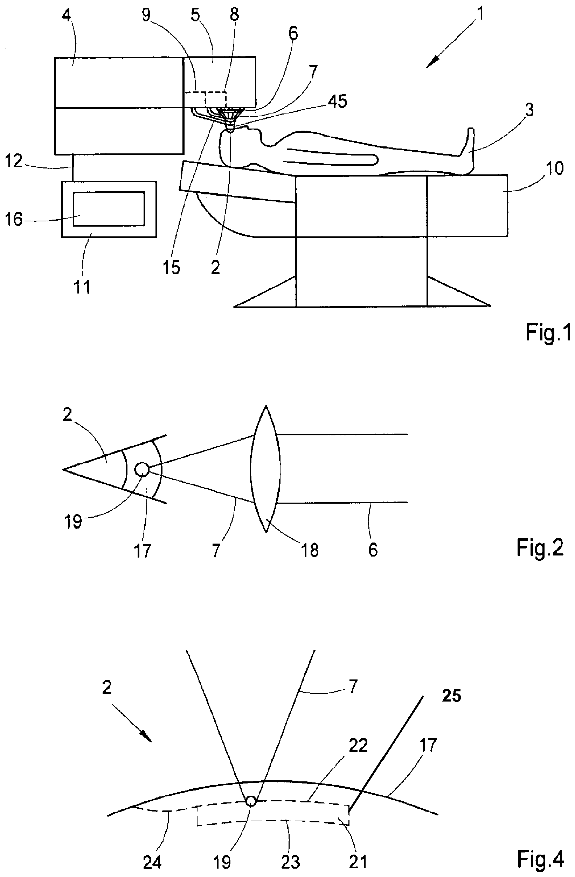

Eine Behandlungsvorrichtung für die Augenchirurgie ist in Fig. 1 dargestellt und mit dem allgemeinen Bezugszeichen 1 versehen. Die Behandlungsvorrichtung 1 ist für die Einbringung von Laserschnitten an einem Auge 2 eines Patienten 3 ausgebildet. Dazu weist die Behandlungsvorrichtung 1 eine Lasereinrichtung 4 auf, die aus einer Laserquelle 5 einen Laserstrahl 6 abgibt, welcher als fokussierter Strahl 7 in das Auge 2 bzw. die Augenhornhaut gerichtet wird. Vorzugsweise ist der Laserstrahl 6 ein gepulster Laserstrahl mit einer Wellenlänge zwischen 300 Nanometer und 10 Mikrometer. Weiter liegt die Pulslänge des Laserstrahls 6 im Bereich zwischen 1 Femtosekunde und 100 Nanosekunden, wobei Pulswiederholraten von 500 bis 50000 Kilohertz und Pulsenergien zwischen 0,01 Mikrojoule und 0,01 Millijoule möglich sind. Die Behandlungsvorrichtung 1 erzeugt somit in der Hornhaut des Auges 2 durch Ablenkung der gepulsten Laserstrahlung eine Schnittfläche. In der Lasereinrichtung 4 bzw. deren Laserquelle 5 ist deshalb dazu noch ein Scanner 8 sowie ein Strahlungsintensitätsmodulator 9 vorgesehen.

Der Patient 3 befindet sich auf einer Liege 10, die optional in drei Raumrichtungen verstellbar ist, um das Auge 2 passend zum Einfall des Laserstrahls 6 auszurichten. In bevorzugter Bauweise ist die Liege 10 motorisch verstellbar. Alternativ ist Patientenliege weniger beweglich und dafür die Behandlungsvorrichtung entsprechend motorisch verstellbar.

Die Ansteuerung kann insbesondere durch ein Steuergerät 11 erfolgen, das grundsätzlich den Betrieb der Behandlungsvorrichtung 1 steuert und dazu über geeignete Datenverbindungen, beispielsweise Verbindungsleitungen 12 mit der Behandlungsvorrichtung verbunden ist. Natürlich kann diese Kommunikation auch über andere Wege, beispielsweise Lichtleiter oder per Funk geschehen. Das Steuergerät 11 nimmt die entsprechenden Einstellungen, Zeitsteuerung an der Behandlungsvorrichtung 1, insbesondere der Lasereinrichtung 4 vor und bewerkstelligt damit entsprechende Funktionen der Behandlungsvorrichtung 1.

Die Behandlungsvorrichtung 1 weist weiter noch eine Fixiereinrichtung 15 auf, welche die Hornhaut des Auges 2 gegenüber der Lasereinrichtung 4 lagefixiert. Diese Fixiereinrichtung 15 kann dabei ein bekanntes Kontaktglas 45 umfassen, an das die Augenhornhaut durch Unterdruck angelegt wird und das der Augenhornhaut eine gewünschte geometrische Form verleiht. Solche Kontaktgläser sind dem Fachmann aus dem Stand der Technik bekannt, beispielsweise aus der DE 102005040338 A1. Der Offenbarungsgehalt dieser Druckschrift wird, soweit die Beschreibung einer Bauform des für die Behandlungsvorrichtung 1 möglichen Kontaktglases 45 betroffen ist, hier vollumfänglich einbezogen.

Andere geänderte oder verbesserte Kontaktglas-Formen könnten auch Vorteile für die Erfindung haben und sollen daher einbezogen werden.

Die Behandlungseinrichtung 1 weist weiterhin eine hier nicht dargestellte Kamera auf, welche durch das Kontaktglas 45 hindurch ein Bild der Augenhornhaut 17 aufnehmen kann. Dabei kann die Beleuchtung für die Kamera sowohl im sichtbaren als auch im infraroten Bereich des Lichtes erfolgen.

Das Steuergerät 11 der Behandlungsvorrichtung 1 weist weiter noch eine Planungseinrichtung 16 auf, die später noch näher erläutert werden wird.

Fig. 2 zeigt schematisch die Wirkungsweise des einfallenden Laserstrahls 6. Der Laserstrahl 6 wird fokussiert und fällt als der fokussierte Laserstrahl 7 in die Hornhaut 17 des Auges 2. Zur Fokussierung ist eine schematisch eingezeichnete Optik 18 vorgesehen. Sie bewirkt in der Hornhaut 17 einen Fokus, in dem die Laserstrahlungsenergiedichte so hoch ist, dass in Kombination mit der Pulslänge der gepulsten Laserstrahlung 6 ein nicht-linearer Effekt in der Hornhaut 17 auftritt. Beispielsweise kann jeder Puls der gepulsten Laserstrahlung 6 im Fokus 19 einen optischen Durchbruch in der Augenhornhaut 17 erzeugen, welche wiederum eine in Fig. 2 nur schematisch angedeutete Plasmablase initiiert. Bei Entstehung der Plasmablase umfasst die Gewebsschichttrennung ein größeres Gebiet als den Fokus 19, obwohl die Bedingungen zur Erzeugung des optischen Durchbruches nur im Fokus 19 erreicht werden. Damit von jedem Laserpuls ein optischer Durchbruch erzeugt wird, muss die Energiedichte, d.h. die Fluence der Laserstrahlung oberhalb eines gewissen, pulslängenabhängigen Schwellwertes liegen. Dieser Zusammenhang ist dem Fachmann beispielsweise aus der DE 69500997 T2 bekannt. Alternativ kann ein gewebetrennender Effekt auch durch gepulste Laserstrahlung erreicht werden, indem mehrere Laserstrahlungspulse in einem Bereich abgegeben werden, wobei sich die Fokus-Spots überlappen. Es wirken dann mehrere Laserstrahlungspulse zusammen, um einen gewebetrennenden Effekt zu erreichen. Die Art der Gewebetrennung, die die Behandlungsvorrichtung 1 einsetzt, ist jedoch für die nachfolgende Beschreibung nicht weiter relevant; wesentlich ist lediglich, dass eine Schnittflächenerzeugung in der Hornhaut 17 des Auges 2 stattfindet.

Die Erfindung verbessert den Druckausgleich im Gebiet der Plasmablasen während sie erzeugt werden und verbessert so die Schnittqualität due Reduzierung der Gewebestörung während des Schneidevorgangs.

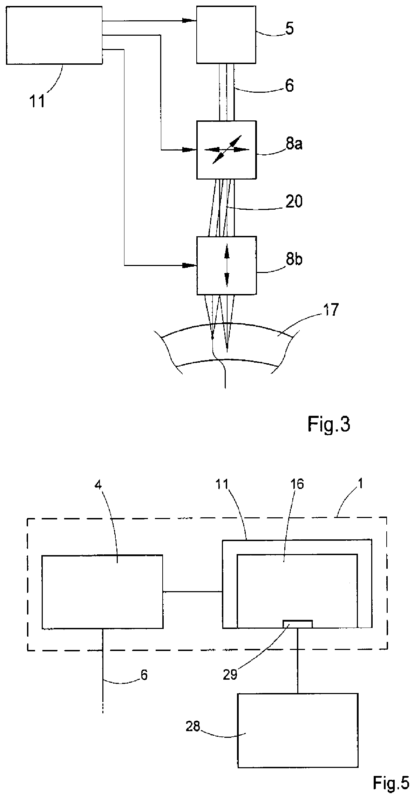

Um eine augenchirurgische Refraktionskorrektur auszuführen, wird mittels der Laserstrahlung 6 aus einem Gebiet innerhalb der Hornhaut 17 ein Hornhautvolumen entfernt, indem dort Gewebeschichten getrennt werden, die das Hornhaut-Volumen isolieren und dann dessen Entnahme ermöglichen. Zur Isolierung des zu entfernenden Hornhaut-Volumens wird z.B. im Falle der gepulst eingebrachten Laserstrahlung die Lage des Fokus 17 der fokussierten Laserstrahlung 7 in der Hornhaut 17 verstellt. Dies ist schematisch in Fig. 3 gezeigt. Die Brechungseigenschaften der Hornhaut 17 werden durch die Entnahme des Volumens gezielt verändert, um so die Refraktionskorrektur zu erreichen. Das Volumen ist deshalb meist linsenförmig und wird als Lentikel bezeichnet.

In Fig. 3 sind die Elemente der Behandlungsvorrichtung 1 nur insoweit eingetragen, als sie zum Verständnis der Schnittflächenerzeugung erforderlich sind. Der Laserstrahl 6 wird, wie bereits erwähnt, in einem Fokus 19 in der Hornhaut 19 gebündelt, und die Lage des Fokus 19 in der Hornhaut wird verstellt, so dass zur Schnittflächenerzeugung an verschiedenen Stellen fokussierende Energie aus Laserstrahlungspulsen in das Gewebe der Hornhaut 17 eingetragen wird. Die Laserstrahlung 6 wird von der Laserquelle 5 vorzugsweise als gepulste Strahlung bereitgestellt. Der Scanner 8 ist in der Bauweise der Fig. 3 zweiteilig aufgebaut und besteht aus einem xy-Scanner 8a, der in einer Variante durch zwei im Wesentlichen orthogonal ablenkende Galvanometerspiegel realisiert ist. Der Scanner 8a lenkt den von der Laserquelle 5 kommenden Laserstrahl 6 zweidimensional ab, so dass nach dem Scanner 9 ein abgelenkter Laserstrahl 20 vorliegt. Der Scanner 8a bewirkt somit eine Verstellung der Lage des Fokus 19 im Wesentlichen senkrecht zur Haupteinfallsrichtung des Laserstrahls 6 in der Hornhaut 17. Zur Verstellung der Tiefenlage ist neben dem xy-Scanner 8a im Scanner 8 noch ein z-Scanner 8b vorgesehen, der beispielsweise als verstellbares Teleskop ausgebildet ist. Der z-Scanner 8b sorgt dafür, dass die z-Position der Lage des Fokus 19, d.h. dessen Position auf der optischen Achse des Einfalls verändert wird. Der z-Scanner 8b kann dem xy-Scanner 8a nach- oder vorgeordnet sein. Um die LIRIC-Struktur zu erzeugen kann die Laserquelle 5 bzgl. Wellenlänge und/oder Leistung umschaltbar ausgeführt sein. Die LIRIC-Struktur wird vorzugsweise bei 405nm Wellenlänge und einer Leistung von 0,01 bis 2 nJ eingeschrieben, während das Einbringen von Schnitten bei 1043 nm Wellenlänge und mit einer Leistung von 50-250 nJ erfolgt. Diese Umschaltung wird durch das Steuergerät 11 bewirkt.

Für das Funktionsprinzip der Behandlungsvorrichtung 1 ist die Zuordnung der einzelnen Koordinaten zu dem Raumrichtungen nicht wesentlich, genau so wenig, dass der Scanner 8a um zueinander rechtwinklige Achsen ablenkt. Vielmehr kann jeder Scanner verwendet werden, der in der Lage ist, den Fokus 19 in einer Ebene zu verstellen, in der die Einfallsachse der optischen Strahlung nicht liegt. Weiter können auch beliebige nichtkartesische Koordinatensystem zur Ablenkung bzw. Steuerung der Lage des Fokus 19 verwendet werden. Beispiele dafür sind Kugelkoordinaten oder zylindrische Koordinaten. Die Steuerung der Lage des Fokus 19 erfolgt mittels der Scanner 8a, 8b unter Ansteuerung durch das Steuergerät 11, das entsprechende Einstellungen an der Laserquelle 5, dem (in Fig. 3 nicht gezeigten) Modulator 9 sowie dem Scanner 8 vornimmt. Das Steuergerät 11 sorgt für einen geeigneten Betrieb der Laserquelle 5 sowie die hier exemplarisch geschilderte dreidimensionale Fokusverstellung, so dass letztendlich eine Schnittfläche ausgebildet wird, die ein bestimmtes Hornhaut-Volumen isoliert, das zur Refraktionskorrektur entfernt werden soll.

Die Steuereinrichtung 11 arbeitet nach vorgegebenen Steuerdaten, welche beispielsweise bei der hier lediglich exemplarisch geschilderten Lasereinrichtung 4 als Zielpunkte für die Fokusverstellung vorgegeben sind. Die Steuerdaten sind in der Regel in einem Steuerdatensatz zusammengefasst. Dies ergibt geometrische Vorgaben für die auszubildende Schnittfläche, beispielsweise die Koordinaten der Zielpunkte als Muster vor. Der Steuerdatensatz enthält dann in dieser Ausführungsform auch konkrete Stellenwerte für den Fokuslagenverstellmechanismus, z.B. für den Scanner 8.

Die Erzeugung der Schnittfläche mit der Behandlungsvorrichtung 1 ist exemplarisch in Fig. 4 gezeigt. Ein Hornhaut-Volumen 21 in der Hornhaut 17 wird durch Verstellung des Fokus 19, in dem der fokussierte Strahl 7 gebündelt ist, isoliert. Dazu werden Schnittflächen ausgebildet, die hier exemplarisch als anteriore Flap-Schnittfläche 22 sowie als posteriore Lentikel-Schnittfläche 23 ausgebildet sind. Diese Begriffe sind hier lediglich exemplarisch zu verstehen und sollen den Bezug auf das herkömmliche LASIK- oder FLEx-Verfahren herstellen, für das die Behandlungsvorrichtung 1, wie bereits geschildert, ebenfalls ausgebildet ist. Wesentlich ist hier lediglich, dass die Schnittflächen 22 und 23 sowie der umlaufende Randschnitt 25, welcher die Schnittflächen 22 und 23 an deren Rändern zusammenführen, das Hornhaut-Volumen 21 isolieren. Durch einen Öffnungsschnitt 24 kann weiter eine das Hornhaut-Volumen 21 anterior begrenzende Hornhaut-Lamelle abgeklappt werden, so dass das Hornhaut-Volumen 21 entnehmbar ist.

Alternativ kann das SMILE-Verfahren eingesetzt werden, bei der das Hornhautvolumen 21 durch einen kleinen Öffnungsschnitt entnommen wird, wie das in der DE 10 2007 019813 A1 beschrieben ist.

Fig. 5 zeigt schematisch die Behandlungsvorrichtung 1, und anhand ihr soll die Bedeutung der Planungseinrichtung 16 näher erläutert werden. Die Behandlungsvorrichtung 1 weist in dieser Variante mindestens zwei Einrichtungen oder Module auf. Die bereits geschilderte Lasereinrichtung 4 gibt den Laserstrahl 6 auf das Auge 2 ab. Der Betrieb der Lasereinrichtung 4 erfolgt dabei, wie bereits geschildert, voll automatisch durch das Steuergerät 11, d.h. die Lasereinrichtung 4 startet auf ein entsprechendes Startsignal hin die Erzeugung und Ablenkung des Laserstrahls 6 und erzeugt dabei Schnittflächen, die auf die beschriebene Art und Weise aufgebaut sind,. Die für den Betrieb erforderlichen Steuersignale empfängt die Lasereinrichtung 5 vom Steuergerät 11, dem zuvor entsprechende Steuerdaten bereitgestellt wurden. Dies erfolgt mittels der Planungseinrichtung 16, die in Fig. 5 lediglich exemplarisch als Bestandteil des Steuergeräts 11 gezeigt ist. Natürlich kann die Planungseinrichtung 16 auch eigenständig ausgebildet sein und drahtgebunden oder drahtlos mit der Steuereinrichtung 11 kommunizieren. Wesentlich ist dann lediglich, dass ein entsprechender Datenübertragungskanal zwischen der Planungseinrichtung 16 und dem Steuergerät 11 vorgesehen ist.

Die Planungseinrichtung 16 erzeugt einen Steuerdatensatz, der dem Steuergerät 11 zur Ausführung der augenchirurgischen Refraktionskorrektur zur Verfügung gestellt wird. Dabei verwendet die Planungseinrichtung Messdaten über die Hornhaut des Auges. Diese Daten stammen in der hier beschriebenen Ausführungsform aus einer Messeinrichtung 28, die das Auge 2 des Patienten 2 zuvor vermessen hat. Natürlich kann die Messeinrichtung 28 auf beliebige Art und Weise ausgebildet sein und die entsprechenden Daten an die Schnittstelle 29 der Planungseinrichtung 16 übermitteln.

Die Planungseinrichtung unterstützt nun den Bediener der Behandlungsvorrichtung 1 bei der Festlegung der Schnittfläche zur Isolierung des Hornhaut-Volumens 21. Dies kann bis zu einer vollautomatischen Festlegung der Schnittflächen gehen, die beispielsweise dadurch bewirkt werden kann, dass die Planungseinrichtung 16 aus den Messdaten das zu entnehmende Hornhaut-Volumen 21 ermittelt, dessen Begrenzungsflächen als Schnittflächen definiert und daraus entsprechende Steuerdaten für das Steuergerät 11 erzeugt. Am anderen Ende des Automatisierungsgrades kann die Planungseinrichtung 16 Eingabemöglichkeiten vorsehen, an denen ein Benutzer die Schnittflächen in Form von geometrischen Parametern etc. eingibt. Zwischenstufen sehen Vorschläge für die Schnittflächen vor, welche die Planungseinrichtung 16 automatisch generiert und die von einem Bearbeiter dann modifizierbar sind. Grundsätzlich können all diejenigen Konzepte, die im vorstehend allgemeineren Beschreibungsteil bereits erläutert wurden, hier in der Planungseinrichtung 16 zur Anwendung kommen.

Um eine Behandlung durchzuführen, erzeugt die Planungseinrichtung 16 Steuerdaten für die Schnittflächenerzeugung, die dann in der Behandlungsvorrichtung 1 verwendet werden.

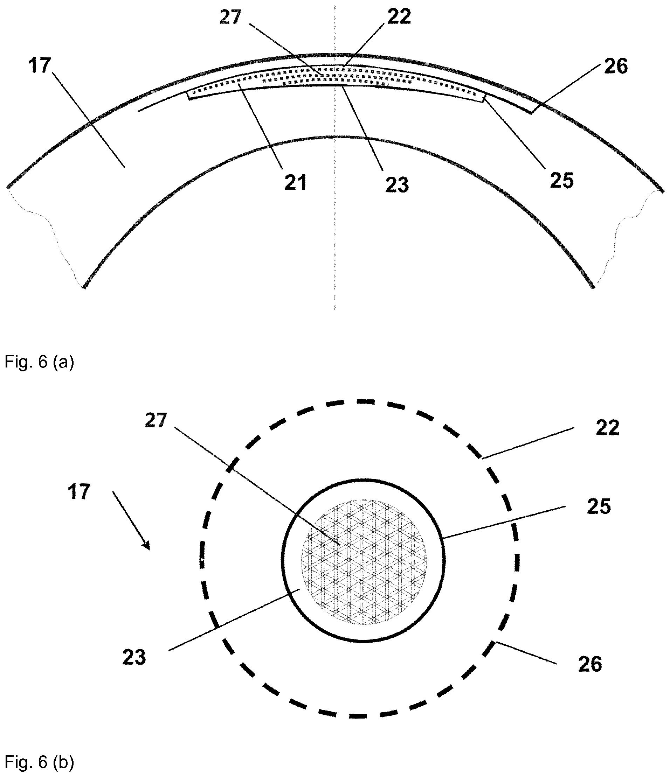

Fig. 6a zeigt eine Schemadarstellung eines Hornhautquerschnitts mit einer LIRIC-Struktur 27 und der Schnittgeometrie des die LIRIC-Struktur einschließenden Lentikels. Die Hornhaut 17 weist einen anterioren Cap-Schnitt 22 mit einem Öffnungsschnitt 26 auf. Der posteriore Lentikelschnitt 23 isolierte das Lentikelvolumen 21, welches durch den Öffnungsschnitt 26 entnommen werden kann. Wenn die Lentikel-Struktur zusammen mit der LIRIC-Struktur erzeugt wird kann auch auf die Erzeugung des Öffnungsschnitts 26 zu diesem Zeitpunkt verzichtet werden und dieser erst bei Notwendigkeit der Entnahme des Lentikelvolumens 21 mit der LIRIC-Struktur 27 eingebracht wird.

Fig. 6b zeigt eine Draufsicht auf die in Fig. 6a dargestellte Hornhaut. Die LIRIC-Struktur 27 wird vollständig von dem durch Cap-Schnitt 22, Lentikelschnitt 23, Randschnitt 25 begrenzten Lentikelvolumen 21umschlossen, ein Öffnungsschnitt ist hier nicht dargestellt, weil er bspw. bei der präventive Enhancement-Struktur nicht sofort erstellt werden muss.

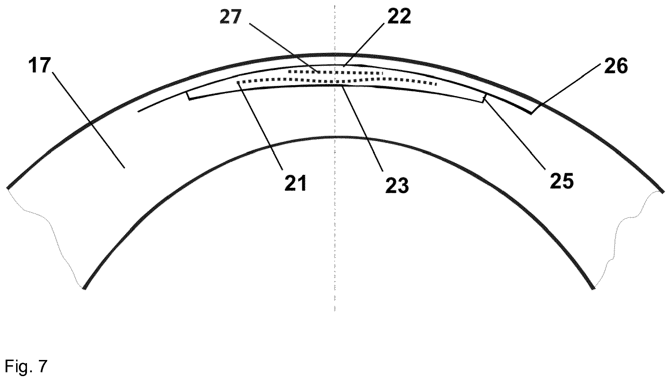

Fig. 7 zeigt eine Schemadarstellung eines Hornhautquerschnitts ähnlich Fig. 6a mit einer anderen LIRIC-Struktur 27 und der Schnittgeometrie des die LIRIC-Struktur einschließenden Lentikels. Hier weist die LIRIC-Struktur 27 in sich eine 3-Dimensionale Verteilung der Brechzahlmodifikation auf.

Zusätzlich sei noch angemerkt, dass die Behandlungsvorrichtung 1 bzw. die Planungseinrichtung 16 natürlich auch die Durchführung des zuvor allgemein erläuterten Verfahrens konkret realisiert.

Eine weitere Ausführungsform der Planungseinrichtung besteht in Form eines Computerprogramms bzw. eines entsprechenden Datenträgers mit einem Computerprogramm, der die Planungseinrichtung auf einem entsprechenden Computer realisiert, so dass die Eingabe der Messdaten über geeignete Datenübertragungsmittel an den Computer erfolgt und die Steuerdaten von diesem Computer an das Steuergerät 11 übertragen werden, wozu wiederum dem Fachmann bekannte Datenübertragungsmittel in Frage kommen.

Während die Erfindung im Detail in den Zeichnungen und der vorstehenden Beschreibung dargestellt wurde, sind die Darstellung und Beschreibung als veranschaulichend oder beispielhaft und nicht einschränkend anzusehen. Es versteht sich, dass Änderungen und Modifikationen durch den Fachmann innerhalb des Umfangs der folgenden Ansprüche vorgenommen werden können. Insbesondere umfasst die vorliegende Erfindung weitere Ausführungsformen mit einer beliebigen Kombination von Merkmalen unterschiedlicher oben und unten beschriebenen Ausführungsformen.

## Patentansprüche

1. Planungseinrichtung zum Erzeugen von Steuerdaten für eine Behandlungsvorrichtung (1) zur Augenchirurgie, die mittels einer Lasereinrichtung (4) zumindest eine Schnittfläche (22, 23) in der Hornhaut erzeugt, wobei die Planungseinrichtung (16) Berechnungsmittel zum Festlegen der Hornhaut-Schnittflächen (22,23) aufweist,
wobei die Berechnungsmittel die Hornhaut-Schnittflächen (22, 23, 25) basierend auf den Daten einer LIRIC-Struktur (27) und vorzugsweise einer Refraktionskorrektur festlegen, und für die Hornhaut-Schnittflächen (22, 23) einen Steuerdatensatz zur Ansteuerung der Lasereinrichtung (4) erzeugen,
**gekennzeichnet dadurch, dass** die Berechnungsmittel die Hornhaut-Schnittflächen (22, 23, 25) so bestimmen, dass die LIRIC-Struktur (27) von den Schnittflächen (22, 23, 25) umschlossen wird.

2. Planungseinrichtung nach Anspruch 1, **gekennzeichnet dadurch, dass** die Hornhaut-Schnittflächen (22, 23, 25) ein Lentikel (21) isolieren, welches die LIRIC-Struktur (27) enthält.

3. Behandlungsvorrichtung zur Augenchirurgie, die
- eine Lasereinrichtung (4) aufweist, welche mittels Laserstrahlung gemäß Steuerdaten zumindest eine Schnittfläche (22, 23) in der Hornhaut erzeugt, und
- eine Planungseinrichtung (16) zum Erzeugen der Steuerdaten nach Anspruch 1 aufweist, wobei die Planungseinrichtung (16) die Hornhaut-Schnittflächen (22, 23, 25) so bestimmt, dass die LIRIC-Struktur (27) von den Schnittflächen (22, 23, 25) umschlossen wird.

4. Behandlungsvorrichtung nach Anspruch 3, **gekennzeichnet dadurch, dass** die Hornhaut-Schnittflächen (22, 23, 25) ein Lentikel (21) isolieren, welches die LIRIC-Struktur (27) enthält.

5. Verfahren zum Erzeugen von Steuerdaten für eine Behandlungsvorrichtung zur Augenchirurgie, die mittels einer Lasereinrichtung zumindest eine Schnittfläche in der Hornhaut erzeugt, wobei das Verfahren durch folgende Schritte gekennzeichnet ist: Bereitstellen von Hornhaut-Daten, basierend auf Daten einer LIRIC-Struktur und vorzugsweise einer Refraktionskorrektur, Festlegen der Hornhaut-Schnittflächen, und Erzeugen eines Steuerdatensatzes für die Hornhaut-Schnittflächen zur Ansteuerung der Lasereinrichtung, wobei die Hornhaut-Schnittflächen so bestimmt werden, dass die LIRIC-Struktur von den Schnittflächen umschlossen wird.

6. Verfahren nach Anspruch 5, **gekennzeichnet dadurch, dass** die Hornhaut-Schnittflächen ein Lentikel isolieren, welches die LIRIC-Struktur enthält.

7. Computerprogrammprodukt mit Programm-Code der bei Ausführung auf einem Computer das Verfahren nach einem der Ansprüche 5 oder 6 ausführt.

8. Datenträger mit einem Computerprogrammprodukt nach Anspruch 7.

## Claims

1. Planning device for producing control data for an ophthalmic surgery treatment apparatus (1) which produces at least one cut surface (22, 23) in the cornea by means of a laser device (4), the planning device (16) comprising calculation means for defining the corneal cut surfaces (22, 23), the calculation means defining the corneal cut surfaces (22, 23, 25) on the basis of the data of a LIRIC structure (27) and preferably a refractive correction, and producing, for the corneal cut surfaces (22, 23), a control data record for controlling the laser device (4),
**characterized in that** the calculation means determine the corneal cut surfaces (22, 23, 25) in such a way that the LIRIC structure (27) is enclosed by the cut surfaces (22, 23, 25).

2. Planning device according to Claim 1, **characterized in that** the corneal cut surfaces (22, 23, 25) isolate a lenticule (21) containing the LIRIC structure (27).

3. Ophthalmic surgery treatment apparatus comprising
- a laser device (4) which, in accordance with control data, produces at least one cut surface (22, 23) in the cornea by means of laser radiation, and
- a planning device (16) for producing the control data according to Claim 1, the planning device (16) determining the corneal cut surfaces (22, 23, 25) in such a way that the LIRIC structure (27) is enclosed by the cut surfaces (22, 23, 25).

4. Treatment apparatus according to Claim 3, **characterized in that** the corneal cut surfaces (22, 23, 25) isolate a lenticule (21) containing the LIRIC structure (27).

5. Method for producing control data for an ophthalmic surgery treatment apparatus which produces at least one cut surface in the cornea by means of a laser device, the method being **characterized by** the following steps: providing corneal data, based on data of a LIRIC structure and preferably a refractive correction, defining the corneal cut surfaces and producing a control data record to control the laser device for the corneal cut surfaces, with the corneal cut surfaces being determined in such a way that the LIRIC structure is enclosed by the cut surfaces.

6. Method according to Claim 5, **characterized in that** the corneal cut surfaces isolate a lenticule containing the LIRIC structure.

7. Computer program product having program code which, upon execution on a computer, carries out the method according to either of Claims 5 and 6.

8. Data medium having a computer program product according to Claim 7.

## Revendications

1. Dispositif de planification permettant de générer des données de commande pour un dispositif de traitement (1) de chirurgie ophtalmique qui génère au moyen d'un dispositif laser (4) au moins une surface de coupe (22, 23) dans la cornée, le dispositif de planification (16) présentant des moyens de calcul servant à définir les surfaces de coupe de cornée (22, 23), les moyens de calcul définissant les surfaces de coupe de cornée (22, 23, 25) sur la base des données d'une structure LIRIC (27) et de préférence d'une correction de réfraction, et générant pour les surfaces de coupe de cornée (22, 23) un jeu de données de commande servant à piloter le dispositif laser (4),
**caractérisé en ce que** les moyens de calcul déterminent les surfaces de coupe de cornée (22, 23, 25) de telle sorte que la structure LIRIC (27) est entourée par les surfaces de coupe (22, 23, 25).

2. Dispositif de planification selon la revendication 1, **caractérisé en ce que** les surfaces de coupe de cornée (22, 23, 25) isolent une lenticule (21) qui contient la structure LIRIC (27).

3. Dispositif de traitement de chirurgie ophtalmique qui
- présente un dispositif laser (4) qui génère au moyen d'un rayonnement laser selon des données de commande au moins une surface de coupe (22, 23) dans la cornée, et
- un dispositif de planification (16) permettant de générer les données de commande selon la revendication 1, le dispositif de planification (16) déterminant les surfaces de coupe de cornée (22, 23, 25) de telle sorte que la structure LIRIC (27) est entourée par les surfaces de coupe (22, 23, 25).

4. Dispositif de traitement selon la revendication 3, **caractérisé en ce que** les surfaces de coupe de cornée (22, 23, 25) isolent une lenticule (21) qui contient la structure LIRIC (27).

5. Procédé permettant de générer des données de commande pour un dispositif de traitement de chirurgie ophtalmique qui génère au moyen d'un dispositif laser au moins une surface de coupe dans la cornée, le procédé étant **caractérisé par** les étapes suivantes consistant à : fournir des données de cornée sur la base de données d'une structure LIRIC et de préférence d'une correction de réfraction, définir les surfaces de coupe de cornée, et générer un jeu de données de commande pour les surfaces de coupe de cornée pour piloter le dispositif laser, les surfaces de coupe de cornée étant déterminées de telle sorte que la structure LIRIC est entourée par les surfaces de coupe.

6. Procédé selon la revendication 5, **caractérisé en ce que** les surfaces de coupe de cornée isolent une lenticule qui contient la structure LIRIC.

7. Produit de programme informatique comprenant du code programme qui, lorsqu'il est exécuté sur un ordinateur, exécute le procédé selon l'une quelconque des revendications 5 ou 6.

8. Support de données comprenant un produit de programme informatique selon la revendication 7.
